# EUROPÄISCHE PATENTSCHRIFT

(11) **EP 2 144 527 B1**
(45) Veröffentlichungstag und Bekanntmachung des Hinweises auf die Patenterteilung: **02.07.2014**
(21) Anmeldenummer: 08716618.7
(22) Anmeldetag: 19.03.2008
(51) Int. Cl.: A45D 34/04, A61K 8/04, B65D 47/06, B65D 51/14, A61Q 15/00

(54) **APPLIKATOR MIT EINER AUSGABEÖFFNUNGSPLATTE FÜR OPTISCH ATTRAKTIVE ANTITRANSPIRANTE FORMULIERUNGEN**
APPLICATOR WITH A DISPENSING OPENING PLATE FOR OPTICALLY ATTRACTIVE ANTIPERSPIRANT FORMULATIONS
APPLICATEUR AVEC UNE PLAQUE À OUVERTURES DE DISTRIBUTION POUR PRÉPARATIONS ANTI-TRANSPIRANTES VISUELLEMENT ATTRAYANTES

(30) Priorität: 09.05.2007 DE 102007022255
(43) Veröffentlichungstag der Anmeldung: 20.01.2010
(73) Patentinhaber: Beiersdorf AG, 20253 Hamburg (DE)
(72) Erfinder: STANGE, Klaus-Peter, 21465 Wentorf (DE); RUESTER, Stefan, 22607 Hamburg (DE); BIEL, Stefan, 21077 Hamburg (DE); RIPKE, Sabine, 22527 Hamburg (DE)
(86) Internationale Anmeldenummer: PCT/EP2008/002180
(87) Internationale Veröffentlichungsnummer: WO 2008/138421

(56) Entgegenhaltungen:
- GB-A- 773 761
- US-A- 5 813 785
- US-A1- 2002 121 530
- US-A1- 2004 232 169

## Beschreibung

Die Erfindung betrifft eine Ausgabeöffnungsplatte für Spenderbehältnisse, die zur Applikation von kosmetischen Antitranspirant-Formulierungen mit stabilisierten Partikeln geeignet sind.

Vor allem aus ästhetischen Gründen werden transparente und transluzente Produkte von vielen Verbrauchern bevorzugt. Transparente Formulierungen kommen so z. B. häufig als Deo oder Antitranspirant (AT) zum Einsatz. Häufig werden diese Zubereitungen mit Partikeln, sogenannten Beads, angereichert. Zubereitungen solcher Art, die frei schwebende Partikel enthalten, sind zum Beispiel aus der DE 10200505551 A, der US 6,793,915 B und US 2004/022 88 86 A bekannt.

Die Herstellung von kosmetischen Zubereitungen, die frei schwebende Partikel enthalten, ist somit dem Fachmann geläufig und stellt keinerlei Hindernis dar.

In der US 2004/023169 wird ein Behältnis mit Ausgabeöffnung beschrieben, das auch für partikelhaltige kosmetische Zubereitungen geeignet ist.

Die in die kosmetischen Zubereitungen eingearbeiteten Partikel können unterschiedlichster Art sein, z,B. homogen oder heterogen aufgebaut, in Form von wirkstoffhaltigen Kapseln oder nur zur optischen Attraktivitätssteigerung der Zubereitung.

In allen Fällen müssen die Partikel jedoch eine gewisse Härte bzw. Festigkeit aufweisen, damit sie nicht bereits im Produktionsprozess der Zubereitung zerstört bzw. aufgebrochen werden. Dies bedeutet jedoch auch, dass sich die Partikel nur durch eine gewisse Krafteinwirkung bei der Applikation verreiben bzw. aufbrechen und mehr oder weniger homogen, in Mischung mit der restlichen, umgebenden Zubereitung, verteilen lassen.

Bei Duschprodukten stellt dies im Allgemeinen kein Hindernis dar, da die Applikation auf dem Körper im Allgemeinen mit der Hand erfolgt, in die eine gewisse Menge an Zubereitung aus einer Flasche appliziert wird und die dann zur Applikation auf dem restlichen Körper mit gewissen Druck über diesen geführt wird.

Bei Deoprodukten erfolgt im Allgemeinen keine direkte Applikation der Zubereitung auf die zu desodorierenden Köperstellen mit der Hand, sondern mit zur Applikation geeigneten Applikatoren. Es hat sich herausgestellt, das die aus dem Stand der Technik bekannten Applikatoren -wie z.B. Spenderstifte mit Spindeltrieb - für Zubereitungen, die keine frei schwebenden Partikel, enthalten wunderbar funktionieren. Geeignete Spender sind bereits aus den Patenanmeldungen US 58 333 82 von Curtis Helene Ind., EP 031 21 65 von Procter & Gamble, EP 111 37 35 von Unilever, DE 690 32 947 von Gilette und DE 199 21 662 von Henkel KGaA bekannt. Solcherlei Spender weisen im Allgemeinen eine Ausgabeöffnungsplatte auf, die einteilig oder mehrteilig mit dem restlichen Spendergehäuse verbunden sein kann und ein oder mehrere Ausgabeöffnungen trägt. Die Ausgabeöffnungsplatte ist in der Regel gewölbt und/oder abgerundet ausgebildet, so das ein leichtes Gleiten über die für zur Applikation vorgesehene Hautareale, insbesondere die Achselhöhle, möglich ist. Wichtig ist es, das auch bei stärkerem Applikationsdruck keine Verletzungen in der Applikationszone auftreten.

Bei Zubereitungen, die frei schwebende Partikel enthalten, erfolgt jedoch eine mangelhafte Verreibung der Partikel, so dass diese störend im Ganzen auf der Haut zurückbleiben. Es hat sich herausgestellt, das hierfür die im Gegensatz zur Handfläche glatte Oberfläche der Applikatoren maßgebllch zur mangelhaften Verreibung beiträgt, da die Partikel nur zwischen bei der Applikatoroberfläche und der Haut 'rollen'. Unter Partikel im Sinne dieser Anmeldung sind insbesondere Feststoffe und Flüssigkeiten, beispielsweise Feststoffkömer oder Flüssigkeitstropfen zu verstehen, welche bevorzugt eine Größe von 200 nm bis zu mehreren Milimetern, bevorzugt zwischen 0,5 mm und 6 mm, aufweisen.

Erschwerend kommt hinzu, dass Partikel, die beim Verreiben des Produkts auf der Hautoberfläche in der Austrittsöffnung ruhen und so keinerlei Scherung durch Verreiben ausgesetzt sind, beim Abheben des Applikators von der Haut durch den aufgetragenen Produktfilm auf der Haut als intaktes Partikel verbleiben.

Auch kann bei herkömmlichen Ausgabeöffnungen ein weiteres die Applikation erschwerendes Phänomen auftreten: Bereits ausgegebene Partikel werden bei der Verteilung des Füllgutes auf der Haut wieder in die Austrittsöffnungen zurückgedrückt (,aufgesammelt'), so das sich die Menge der effektiv aufgetragenen Partikel stark verringert.

Dieser 'Staubsaugereffekt' wird erfolgreich durch die Verwendung der erfinderischen Ausgabeöffnungsplatten bzw. Ausgabeöffnungen verhindert.

Aufgabe der vorliegenden Erfindung ist es, eine Ausgabeöffnungsplatte zur Verfügung zu stellen, die sich für Spenderbehältnisse eignet, mit denen kosmetische Zubereitungen aufweisend mindestens eine Art von frei schwebenden Partikeln appliziert werden können.

Es war überraschend und für den Fachmann nicht vorauszusehen war, dass Ausgabeöffnungsplatten gemäß dem Hauptanspruch, deren Ausgabeöffnung(en) auf der Innenseite mindestens einen Zahn oder Zinken aufweisen, welcher in den freien Durchgang hineinragt, die Verreibung von in kosmetische Zubereitungen enthaltenen Partikel unterstützen.

Wichtig ist hierbei, dass der Durchmesser der Ausgabeöffnungen so gewählt wird, dass der Hauptanteil der Partikel durch diese Öffnungen hindurch gelangen kann ohne die Öffnung zu verstopfen. Beim Durchtritt durch die Ausgabeöffnung werden die Partikel durch die in die Öffnung hineinragenden Zähne oder Zinken eingekerbt bzw. gespalten, wodurch die kugelrunde Form verloren geht. Die nicht mehr kugelrunden Partikel lassen sich anschließend beim Auftragsvorgang leicht verreiben.

Durch Einsatz der erfindungsgemäßen Ausgabeöffnungesplatten ist in der Regel eine rückstandsfreie Verreibung der Partikel gewährleistet.

Erfindungsgemäß ist es, wenn das Größenverhältnis des mittleren Durchmesser der Partikel zum Durchmesser der Ausgabeöffnungen im Bereich 1:1 bis 0,5:1 beträgt. Bei einem Durchmesserverhältnis von größer 1,1:1 verstopfen die Ausgabeöffnungen sehr leicht und ein Auftragen von Zubereitung ist nicht oder nur ungenügend möglich.

Vorteilhaft ist es, wenn die Zinken oder Zähne den Ausgabeöffnungsdurchmesser, d.h. den größten freien Durchmesser, so verringern, das der kleinste freie Durchmesser unter Berücksichtigung der Zähne oder Zinken um 10 bis 50 % geringer ausfällt.

Als ganz besonders vorteilhaft Ausgabeöffnungsdurchmesser hat sich ein Durchmesser von 1,5 mm ± 0,5 mm herausgestellt., wobei sich der Ausgabeöffnungsdurchmesser immer nach dem größten freien Durchmesser, ohne Berücksichtigung der Zähne oder Zinken, bemisst und die hineinragenden Zähne Durchlass auf 1 mm ±0,5 mm verringern.

Bei einer weiteren besonders vorteilhafte Ausführungsform, sind die Zähne unterhalb der äußeren Gridoberfläche positioniert, was einen entscheidenden Einfluss auf den Erfolg der Verreibung der Partikel auf der Haut hat. Insbesondere vorteilhaft scheint die Positionierung der Zähne oder Zinken in den Ausgabeöffnungen zu sein, wenn diese um 10 bis 75 % des mittleren Partikeldurchmessers in die Ausgabeöffnung hinein nach innen (von der äußeren Gridoberfläche aus gesehen) versetzt werden.

Im Sinne der Erfindung ist es auch, die Ausgabeöffnungen nicht kreisrund auszuführen, sondern einen ellipsoiden Querschnitt zu verwenden.

Eine weitere verbesserte Ausführungsform weist Ausgabeöffnungen auf, deren Grundform nicht zylindrisch ist, sondern eine leicht von der zylindrischen Form abweichende, kegelstumpfförmige, Ausprägung aufweist, wobei der größere Durchmesser auf der Oberflächenseite auftritt, die zum Inneren des Behältnisses zeigt.

Des Weiteren ist es vorteilhaft, den nach außen weisenden Rand der Ausgabeöffnung abzurunden oder mit einer Phase oder Einsenkung zu versehen. Die Gleiteigenschaften auf der Haut werden dadurch erheblich verbessert.

Um eine vollständige Übertragung der Zubereitung von der Applikatoroberfläche auf die Haut zu gewährleisten, ist es zudem vorteilhaft, wenn die Applikatoroberfläche neben den Ausgabeöffnungen keine weiteren Strukturierungen, wie z.B, Rillen oder Nuten, die das Zerreiben der Partikel unterstützen, aufweisen.

Durch die erfindungsgämaßen Ausgabeöffnungsplatten lassen sich die in kosmetischen Zubereitungen enthaltenen Partikel, besonders gut unter Verreibung, applizieren.

Die Fixierung der Ausgabeöffnungsplatte im Applikator, kann durch gebräuchliche Konstruktionen oder Hilfmittel, wie zum Beispiel durch Verrastung, Verkleben, Verschweißen, erfolgen.

Ganz besonders geeignet sind Formulierungen, die die die Partikel in der Formulierung frei schwebend aufweisen, d.h. die Schwerkarft führt auch bei längerer Standzeit zu keinem Absinken der Partikel in der Formulierung.

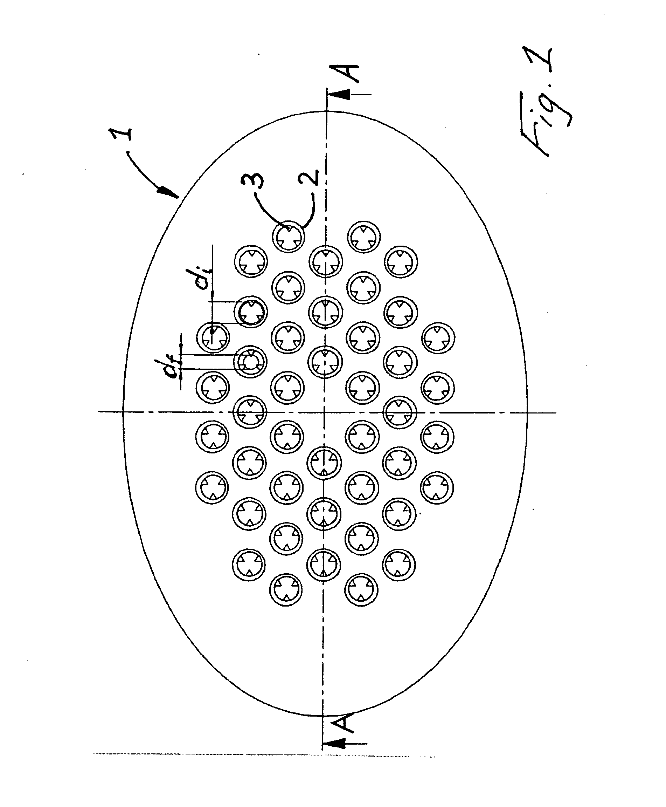

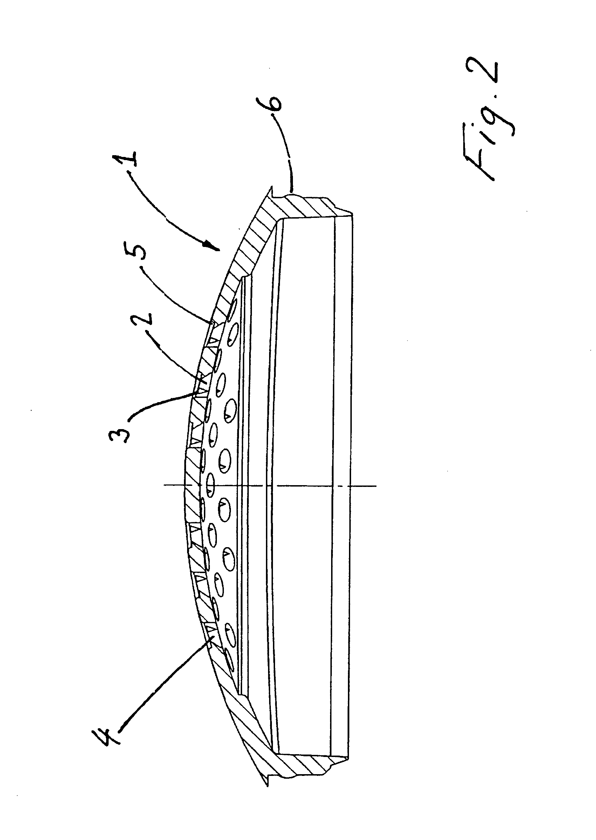

In den Figuren 1 und 2 ist beispielhaft eine Ausgabeöffnungsplatte wiedergegeben, die sich als besonders vorteilhaft herausgestellt hat. Dieses Beispiel soll sich jedoch nicht limitierend auf die Erfindung auswirken, sondern verdeutlichen, welchen Gestaltungsspielraum es gibt.

Figur 1 zeigt die Aufsicht auf eine Ausgabeöffnungsplatte (1), die insgesammt 44 Ausgabeöffnungen (2) aufweist. Jede Ausgabeöffnung weist drei Zähne (3) auf, die sich von der Innenwand der Ausgabeöffnung in das Zentrum der Ausgabeöffnung erstrecken. Der Innendurchmesser dₗ (größter freier Durchmesser) jeder Ausgabeöffnung (2) beträgt 1,5 mm, woduch sich ein freier Querschnitt (kleinster freier Durchmesser unter Berücksichtigung der Zähne oder Zinken) von d_{f} = 1 mm ergibt.

Figur 2 zeigt einen Querschnitt (A-A) der in Figur 1 dargestellten Ausgabeöffnungsplatte (1). Die Zähne (3) sind gegenüber der äußeren Oberfläche der Ausgabeöffnungsplatte um den Abstand (d_{z}) zurückgesetzt. Die Ausgabeöffnungen (2) weisen auf der Aussenseite eine Einsenkung (5), die fast bis auf die Oberkante der Zähne (3) hinabreicht. Die Ausgabeöffnungen (2) sind kegelstumpfförmig (4) ausgebildet. Zur Verankerung der Ausgabeöffnungsplatte im Applikator, weist diese eine umlaufende Rastnut (6) auf.

## Patentansprüche

1. Applikator mit einer Ausgabeöffnungsplatte (1), wobei der Applikator eine kosmetischen Zubereitung enthält und die kosmetische Zubereitung mindestens eine Art von freischwebenden Partikeln enthält, wobei die Ausgabeöffnungsplatte mindestens eine Ausgabeöffnung aufweist und die Ausgabeöffnung(en) (2) ein oder mehrere Zähne oder Zinken (3) aufweisen, die sich von der Innenwand der Ausgabeöffnung in das Zentrum der Ausgabeöffnung erstrecken, **dadurch gekennzeichnet, dass** das Größenverhältnis des mittleren Durchmessers der Partikel zum größten freien Durchmesser der Ausgabeöffnungen im Bereich 1:1 bis 0,5:1 beträgt und dass die Partikel beim Durchtritt durch die Ausgabeöffnungen durch die in die Öffnungen hineinragenden Zähne oder Zinken eingekerbt oder gespalten werden.

2. Applikator nach Anspruch 1, dadurch gekennzeichent, dass der kleinste freie Durchmesser (d) unter Berücksichtigung der Zähne oder Zinken gegenüber der größte freien Durchmesser (dᵢ) um 10 bis 50 % geringer ist.

3. Applikator nach mindestens einem der vorhergehenden Ansprüche, **dadurch gekennzeichnet, dass** die nach innen hineinragenden Zähne den freien Durchmesser (d_{f}) auf 0,5 bis 3 mm verengen.

4. Applikator nach mindestens einem der vorhergehenden Ansprüche, **dadurch gekennzeichnet, dass** die Ausgabeöffnungen einen Durchmesser (dᵢ) von 1 bis 5 mm aufweisen, wobei sich der Ausgabeöffnungsdurchmesser immer nach dem größten freien Durchmesser, ohne Berücksichtigung der Zähne oder Zinken, bemisst.

5. Applikator nach mindestens einem der vorhergehenden Ansprüche, **dadurch gekennzeichnet, dass** die Zähne oder Zinken (3) in den Ausgabeöffnungen so positioniert sind, das sie um einen Abstand (d_{z}), der 10 bis 75 % des mittleren Partikeldurchmessers entspricht, von der äußeren Ausgabeöffnungsplattenoberfläche aus gesehen in die Ausgabeöffnung hinein nach innen versetzt angeordnet sind.

6. Applikator nach mindestens einem der vorhergehenden Ansprüche, **dadurch gekennzeichnet, dass** die Grundform der Ausgabeöffnungen eine kegelstumpfförmige Ausprägung aufweist, wobei der größere Durchmesser auf der Oberflächenseite auftritt, die zum inneren des Behältnisses zeigt.

7. Applikator nach mindestens einem der vorhergehenden Ansprüche, **dadurch gekennzeichnet, dass** Rand der Ausgabeöffnung abgerundet oder mit einer Phase oder Einsenkung (5) zu versehen ist.

8. Verwendung von Applikatoren nach mindestens einem der vorhergehenden Ansprüche zur Applikation von partikelhaltigen kosmetischen Zubereitungen.

9. Verwendung nach Anspruch 8, **dadurch gekennzeichnet, dass** die Partikel einen Durchmesser von 0,5 bis 5 mm aufweisen.

10. Verwendung nach Anspruch 8 oder 9, **dadurch gekennzeichnet, dass** das Größenverhältnis des mittleren Durchmesser der Partikel zum Durchmesser der Ausgabeöffnungen im Bereich 1:1 bis 0,8:1 liegt.

11. Verwendung nach mindestens einem der Ansprüche 8 bis 10, **dadurch gekennzeichnet, dass** das die Zubereitung eine Antitranspirant und/oder Deodorantzubereitung, insbesondere eine gelförmige Antitranspirant und/oder Deodorantzubereitung, ist.

## Claims

1. Applicator having a dispensing-opening plate (1), wherein the applicator contains a cosmetic preparation and the cosmetic preparation contains at least one type of free-floating particles, wherein the dispensing-opening plate has at least one dispensing opening and the dispensing opening(s) (2) has or have one or more teeth or prongs (3) which extend from the inner wall of the dispensing opening into the centre of the dispensing opening, **characterized in that** the ratio of the average diameter of the particles to the largest free diameter of the dispensing openings is in the range from 1:1 to 0.5:1, and **in that** when the particles pass through the dispensing openings, said particles are indented or split by the teeth or prongs projecting into the openings.

2. Applicator according to Claim 1, **characterized in that** the smallest free diameter (d), taking into account the teeth or prongs, is 10 to 50% smaller than the largest free diameter (dᵢ).

3. Applicator according to at least one of the preceding claims, **characterized in that** the inwardly projecting teeth narrow the free diameter (d_{f}) to 0.5 to 3 mm.

4. Applicator according to at least one of the preceding claims, **characterized in that** the dispensing openings have a diameter (dᵢ) of 1 to 5 mm, wherein, the dispensing-opening diameter is always measured according to the largest free diameter, without taking into account the teeth or prongs.

5. Applicator according to at least one of the preceding claims, **characterized in that** the teeth or prongs (3) are positioned in the dispensing openings such as to be arranged in a manner offset inwardly into the dispensing opening, as seen from the outer dispensing-opening-plate surface, by a distance (d_{z}) which corresponds to 10 to 75% of the average particle diameter.

6. Applicator according to at least one of the preceding claims, **characterized in that** the basic shape of the dispensing openings has a frustoconical characteristic, wherein the larger diameter is present on the surface side which is directed towards the interior of the container.

7. Applicator according to at least one of the preceding claims, **characterized in that** the rim of the dispensing opening is rounded or is intended to be provided with a bevel or depression (5).

8. Use of applicators according to at least one of the preceding claims for the application of particle-containing cosmetic preparations.

9. Use according to Claim 8, **characterized in that** the particles have a diameter of 0.5 to 5 mm.

10. Use according to Claim 8 or 9, **characterized in that** the ratio of the average diameter of the particles to the diameter of the dispensing openings is in the range from 1:1 to 0.8:1.

11. Use according to at least one of Claims 8 to 10, **characterized in that** the preparation is an antiperspirant and/or deodorant preparation, in particular a gel-form antiperspirant and/or deodorant preparation.

## Revendications

1. Applicateur comprenant une plaque d'ouverture de distribution (1), l'applicateur contenant une préparation cosmétique et la préparation cosmétique contenant au moins un type de particules librement en suspension, la plaque d'ouverture de distribution présentant au moins une ouverture de distribution et la ou les ouvertures de distribution (2) présentant une ou plusieurs dents ou pointes (3) qui s'étendent depuis la paroi interne de l'ouverture de distribution jusque dans le centre de l'ouverture de distribution, **caractérisé en ce que** le rapport de taille entre le diamètre moyen des particules et le plus grand diamètre libre des ouvertures de distribution est de l'ordre de 1:1 à 0, 5:1 et **en ce que** les particules, lors de leur passage à travers les ouvertures de distribution, sont entaillées ou fendues par les dents ou les pointes pénétrant dans les ouvertures.

2. Applicateur selon la revendication 1, **caractérisé en ce que** le plus petit diamètre libre (d), en tenant compte des dents ou des pointes, est inférieur de 10 à 50 % par rapport au plus grand diamètre libre (d₁).

3. Applicateur selon au moins l'une quelconque des revendications précédentes, **caractérisé en ce que** les dents pénétrant vers l'intérieur réduisent le diamètre libre (d_{f}) à 0,5 à 3 mm.

4. Applicateur selon au moins l'une quelconque des revendications précédentes, **caractérisé en ce que** les ouvertures de distribution présentent un diamètre (dᵢ) de 1 à 5 mm, le diamètre de l'ouverture de distribution étant toujours mesuré en fonction du plus grand diamètre libre, sans tenir compte des dents ou des pointes.

5. Applicateur selon au moins l'une quelconque des revendications précédentes, **caractérisé en ce que** les dents ou les pointes (3) sont positionnées dans les ouvertures de distribution de telle sorte qu'elles soient disposées de manière décalée d'une distance (d_{z}) qui correspond à 10 à 75 % du diamètre moyen des particules, vu depuis la surface extérieure de la plaque d'ouverture de distribution, vers l'intérieur dans l'ouverture de distribution.

6. Applicateur selon au moins l'une quelconque des revendications précédentes, **caractérisé en ce que** la forme de base des ouvertures de distribution présente un gaufrage de forme tronconique, le plus grand diamètre étant situé sur le côté de la surface tourné vers l'intérieur du récipient.

7. Applicateur selon au moins l'une quelconque des revendications précédentes, **caractérisé en ce que** le bord de l'ouverture de distribution est arrondi ou doit être pourvu d'un biseau ou d'une dépression (5).

8. Utilisation d'applicateurs selon au moins l'une quelconque des revendications précédentes pour l'application de préparations cosmétiques contenant des particules.

9. Utilisation selon la revendication 8, **caractérisée en ce que** les particles présentent un diamètre de 0,5 à 5 mm.

10. Utilisation selon la revendication 8 ou 9, **caractérisée en ce que** le rapport de taille du diamètre moyen des particules au diamètre des ouvertures de distribution est compris dans la plage de 1:1 à 0,8:1.

11. Utilisation selon au moins l'une quelconque des revendications 8 à 10, **caractérisée en ce que** la préparation est un anti-transpirant et/ou une préparation de déodorant, en particulier un anti-transpirant et/ou une préparation de déodorant sous forme de gel.
